# EUROPEAN PATENT SPECIFICATION

(11) **EP 1 790 377 B1**
(45) Date of publication and mention of the grant of the patent: **14.04.2010**
(21) Application number: 06022838.4
(22) Date of filing: 02.11.2006
(51) Int. Cl.: A61M 39/22

(54) **Stopcock for medical treatment**
Absperrhahn für medizinischen Behandlung
Robinet d'arrêt pour un traitement médical

(30) Priority: 28.11.2005 JP 2005341693
(43) Date of publication of application: 30.05.2007
(73) Proprietor: Covidien AG, 8212 Neuhausen am Rheinfall (CH)
(72) Inventor: Iguchi, Masanobu, Fukuroi-shi, Shizuoka-ken (JP); Kurimoto, Wataru, Fukuroi-shi, Shizuoka-ken (JP)
(74) Representative: Tomlinson, Edward James

(56) References cited:
- EP-A- 1 459 783
- EP-A2- 0 694 315
- DE-A1- 3 019 426
- DE-A1- 4 232 082
- GB-A- 833 247
- GB-A- 1 506 688
- US-A- 5 284 134
- US-A- 5 356 396
- US-A- 5 549 583
- US-B1- 6 589 197

## Description

### Technical field

The present invention relates to a valve unit also termed a stopcock for medical treatment connected to multiple transfusion tubes or the like used for medical treatment for switching between opening or blocking off individual transfusion tubes.

### Prior art

In the past, prescribed physiological saline, liquid medicine or the like would have been supplied to a patient's body using multiple transfusion tubes, and in such cases opening or blocking off the individual transfusion tubes would have been accomplished using a stopcock for medical treatment. Such stopcocks for medical treatment include three-way stopcocks provided with three branch tubes (refer to Japanese Kokai Patent Application No. Sho 61 [1986]-68045, for example). The stopcock for medical treatment is constituted with a stopcock body and a valve body. The stopcock body is constituted with three branch tubes formed integrally extending toward the outside held at 90° on the outer circumferential surface of a cylindrical chamber part. The valve body is installed inside the chamber part to be able to which can rotate around the axis of the chamber part, and a groove for connecting to any of the three branch tubes is formed on its outer circumferential surface. Therefore, any branch tube can be opened by rotating the valve body in the chamber part to a prescribed angle.

A plastics manifold and valve assembly is described in GB 1 506 688 in which luer connections are integrally moulded with a device housing. US 4,219,021 also describes an arrangement in which a luer connection is integral with a stop-cock valve housing. US 5,549,583 describes a connector for providing at one end a luer connection and at a second end a rotatable snap connection.

### Summary of the invention

Generally, polycarbonate is often used as the material for constituting the stopcock body in such a stopcock for medical treatment because of its outstanding moldability and the fact that liquid leaks do not occur easily when a valve body is used attached to the stopcock body, making high-precision molding possible. However, polycarbonate has the problem that chemical its resistance is unsatisfactory. It is particularly susceptible to alcohol and anti-cancer agents, and when used in contact with liquid medicines that include components such as these, there is the risk of cracks occurring at the connections with other parts. When a material with outstanding chemical resistance, polypropylene, for example, is used to solve this problem, the problem that occurs is that it is difficult to obtain a molded article with good precision. In addition, with the abovementioned stopcock for medical treatment, the inside of the chamber is also exposed to the external air and there is the risk of bacterial growth.

The present invention was devised in consideration of such circumstances. Its objective is to provide a stopcock for medical treatment with outstanding moldability and chemical resistance.

In order to achieve the aforementioned objective, the features in the constitution of a stopcock for medical treatment pertaining to the present invention are that it is a stopcock for medical treatment composed of a stopcock body that is composed of a cylindrical chamber and multiple branch tubes that extend toward the outside from the outer circumferential surface of the aforementioned chamber with the gap between them maintaining a prescribed angle and having a channel that connect to the inside of the aforementioned chamber; and a resin molded body that has a valve body installed inside of the aforementioned chamber which can rotate around the axis of the aforementioned chamber and in which a groove for connecting to any of the channels of the aforementioned multiple branch tubes is formed, and that at least a connection part for connecting to another member in a prescribed branch tube of the aforementioned multiple branch tubes is constituted with a separate member from the other portion that constitutes the aforementioned stopcock body, the portion constituted by the separate member that includes the aforementioned connection part is constituted with a material that has outstanding chemical resistance, and the other portion that constitutes the aforementioned stopcock body is constituted with a material that can be molded with high dimensional precision.

With the stopcock for medical treatment of the present invention constituted as described above, rather than the stopcock body being constituted with a single resin molded body, at least a connection part connected to another member in the prescribed branch tube is constituted with a separate body from the other portion toward the chamber. Therefore, by constituting portions that include a connection part that readily deteriorates in contact with a liquid medicine so that cracks occur with a material with outstanding chemical resistance, cracks and breaks in the stopcock body can be prevented from occurring. By constituting other portions of the stopcock body in which cracks and breaks do not readily occur with a material that can be molded with high dimensional precision, a stopcock body with satisfactory precision can be obtained. The portion constituted by the separate member in this case may be the entirety of the prescribed branch tube or the portion toward the end of the prescribed branch tube could be formed integrally with the chamber and the portion toward the tip could be constituted as a separate member. That is, it is sufficient for the portion, including at least a connection part that requires chemical resistance, to be constituted with a separate member.

The feature in another constitution of a stopcock for medical treatment pertaining to the present invention is that the portion constituted with a separate member is a female Luer part into which the male Luer part of another member is inserted. Because of this, the female Luer part, which has a structure that is easily susceptible to stress from the male Luer part due to the male Luer part of the other member being inserted, and that cracks easily, is constituted with a material with outstanding chemical resistance. For this reason, deterioration of the female Luer part due to the effect of the liquid medicine is suppressed, and it is difficult for cracks to occur even when stress is applied from the male Luer part.

The feature in another constitution of a stopcock for medical treatment pertaining to the present invention is that the connection part for the portion constituted with a separate member and the other portion constituting the stopcock body is connected with a seal material between. An O-ring made of rubber, for example, can be used as the seal material in this case. Even when the stopcock body is constituted with multiple members as for the stopcock for medical treatment of the present invention, by connecting the connection parts with a seal material between them, leaks from the connection parts can be more reliably prevented from occurring.

The features in still another constitution of a stopcock for medical treatment pertaining to the present invention are that the stopcock for medical treatment is constituted with a three-way stopcock that has three branch tubes and that the open part of at least one of the three branch tubes is closed with a rubber plug that can be punctured with a sharp member. A three-way stopcock such as this is normally formed so that the two branch tubes arranged in a straight line sandwiching the chamber part serve as main channels and the other branch tube serves as a coinjection route converging with the main channels. That is, it is constituted so that another liquid medicine or the like from the coinjection route is temporarily or continually mixed into the liquid medicine passing through the main channel. Therefore, its frequency of use is relatively low and, for example, air entering the chamber part and bacterial growth can be prevented by attaching a rubber plug in the branch tube constituting the coinjection route. When a liquid medicine or the like is mixed in from the coinjection path, a sharp member, e.g., a hypodermic needle or a male Luer part, can be inserted and the liquid medicine can be mixed into the main channel through the sharp member.

The features of still another constitution of a stopcock for medical treatment pertaining to the present invention are that the material with outstanding chemical resistance is a polyolefin resin material and that the material that can be molded with high dimensional stability is a polycarbonate. Because of this, dimensional stability is improved, and a stopcock for medical treatment with outstanding chemical resistance in which cracks and breaks do not readily occur can be obtained.

### Brief description of the figures

The present invention will now be described, by way of example only, with reference to the accompanying drawings in which:
Figure 1 shows a stopcock for medical treatment pertaining to an embodiment of the present invention; (a) is a front view, (b) a side view, and (c) a bottom view;
Figure 2 is a cross section at 2-2 in Figure 1 (c);
Figure 3 is a cross section at 3-3 in Figure 1 (a);
Figure 4 is an exploded bottom view showing the stopcock body;
Figure 5 is an exploded cross section showing the stopcock body; and
Figure 6 shows a stopcock for medical treatment pertaining to another embodiment of the present invention; (a) is a front view and (b) is a bottom view.

### Preferred embodiment of the invention

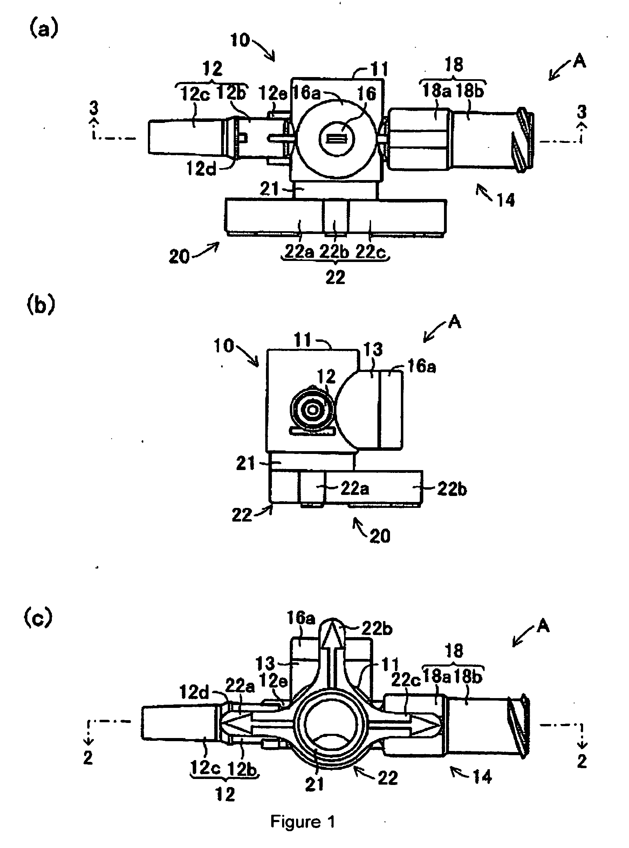

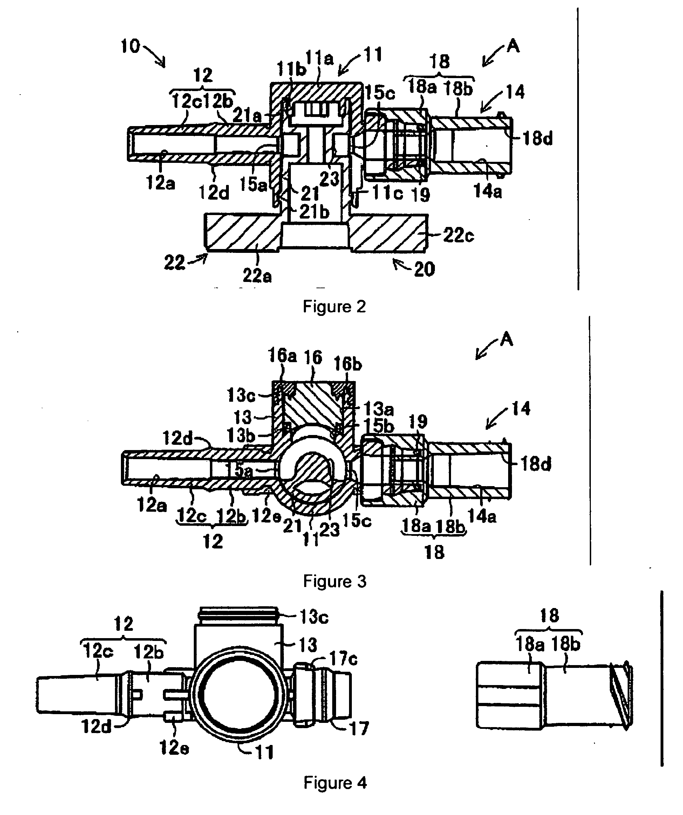

Stopcocks for medical treatment pertaining to the present invention will be explained below in detail with reference to the figures. Figures 1 and 2 show a stopcock for medical treatment (A) that pertains to an embodiment of the present invention. A stopcock for medical treatment (A) is constituted with a stopcock body (10) and a valve body (20). Stopcock body (10) is constituted with a cylindrical chamber (11) that is short in the axial orientation, and branch tubes (12), (13) and (14) that are coupled to the outer circumferential surface of chamber part (11) maintaining an angle of 90°. Chamber (11), as shown in Figure 2, is arranged with the axial orientation vertically and the top end formed into a closed cylindrical shape.

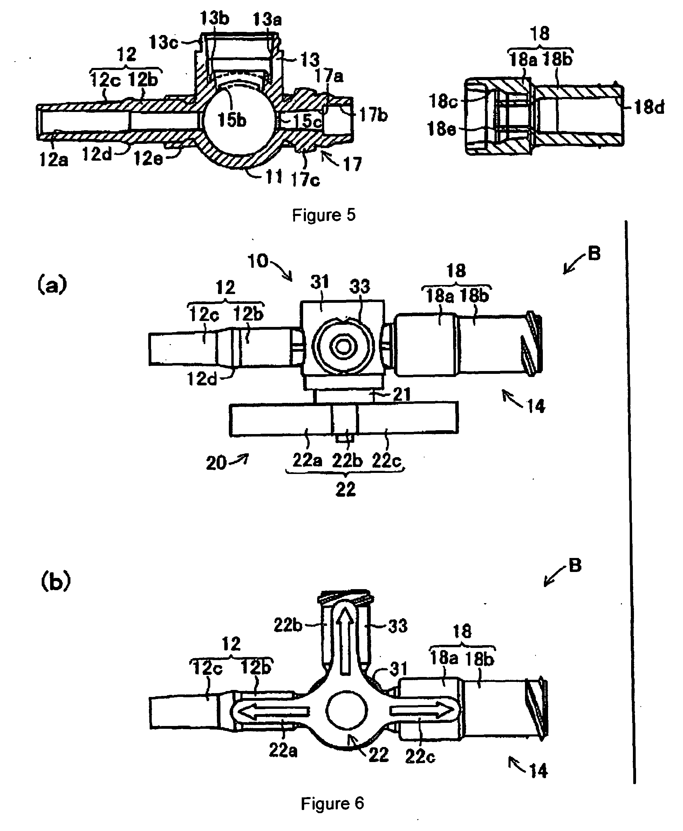

A ring-shaped engaging part (11 b) that extends downward is also formed on the underside of ceiling (11a) that constitutes the top end of chamber (11) provided with a prescribed spacing from inner circumferential surface of chamber part (11). A shallow engaging groove (11c) is also formed in the portion toward the lower end on the inner circumferential surface of chamber (11). Additionally, in the approximate middle, in axial orientation, of chamber (11), connecting holes (15a), (15b) (refer to Figures 3 and 5), and (15c) are formed. Branch tube (12) is furnished for the portion corresponding to connecting hole (15a) in chamber (11), and the inside of chamber (11) and a channel (12a) formed inside branch tube (12) are connected through connecting hole (15a).

The connecting hole (15b) is constituted so that its diameter is set larger than the diameters of connecting holes (15a) and (15c) and a taper is additionally furnished on the inner circumferential surface so that the diameter on the outside is larger than the diameter on the inside. Branch tube (13) is furnished for the portion corresponding to connecting hole (15b) in chamber part (11), and the inside of chamber (11) and channel (13a) formed in branch tube (13) are connected through connecting hole (15b). Note that, with the state in Figure 3, a rubber plug (16), described below, is inserted inside branch tube (13) and channel (13a) is blocked. Branch tube (14) is furnished in the portion corresponding to connecting hole (15c) in chamber (11) and the inside of chamber (11) and channel (14a) formed in branch tube (14) are connected through connecting hole (15c).

Branch tube (12) is formed integrally with chamber (11) and is constituted with base part (12b) toward chamber (11) and a male Luer part (12c) toward the tip formed to be narrower than base end part (12b). Male Luer part (12c) is also formed into a pointed shape such that toward the tip it is narrower than toward base part (12b). A projection (12d) for engaging is also formed circumferentially at the boundary between base part (12b) and male Luer part (12c) on the outer circumferential surface of branch tube (12), and multiple reinforcing ribs (12e) that extend axially from chamber (11) are formed around the axis, maintaining a spacing in the portion on the outer circumferential surface of base (12b) toward chamber (11).

Branch tube (13) is formed into a cylindrical shape that is shorter in the axial orientation and larger in diameter than branch tube (12) and constituted integrally with chamber (11). A taper is furnished in the inner circumferential surface of branch tube (13) so that the diameter toward the opening is larger than the diameter toward chamber (11) and a ring-shaped engaging part (13b) that extends upward, furnished with a prescribed spacing from the inner circumferential surface of branch tube (13), is formed in the portion in branch tube (13) that connects with connecting hole (15b).That is, the diameter of the inner circumferential surface of branch tube (13) is set to be greater than the diameter of the inner circumferential surface of connecting hole (15b), and a level difference is formed between the inner circumferential surface of branch tube (13) and the inner circumferential surface of connecting hole (15b). An engaging part (13b) is formed in the level difference portion.

The portion toward the opening in branch tube (13) is formed thin so that the inside diameter is smaller than the other portion of branch tube (13) and a projection (13c) for engaging is formed circumferentially on the outer circumferential surface of the thin part. Inside branch tube (13), a rubber plug (16) made of natural or synthetic rubber is inserted with the part around the outside of the lower surface engaged in engaging part (13b). The underside of rubber plug (16) blocks connecting hole (15b) and is formed into a curved surface that does not follow the outer circumferential surface of chamber (11) so that it is not squeezed. A slit that penetrates between the inside of chamber (11) and the outside of branch tube (13) is furnished in rubber plug (16), but normally the slit is closed by the elasticity of rubber plug (16).

A lid part (16a) for keeping rubber plug (16) inside branch tube (13) is attached to the open part of branch tube (13). Lid part (16a) is formed approximately into a ring shape in which the center part of the upper surface is open and an engaging recess (16b) that can engage with projection (13c) is formed in the inner circumferential surface at the side. Therefore, lid part (16a) presses the portion around the outside of the upper surface of rubber plug (16) and is attached to the open part of branch tube (13) with engaging recess (16b) engaged with projection (13c).

Branch tube (14), as shown in Figures 4 and 5, is constituted with coupling part (17) formed integrally with chamber part (11) and with a female Luer part (18) serving as the connection part of the present invention removably attached to coupling part (17). The thickness of coupling part (17) is set to around the same as the thickness of base part (12b) of branch tube (12), and the length, in axial orientation, of coupling part (17) is set to around the same length as the length, in axial orientation, of branch tube (13). Connecting hole (15c) connects to the portion inside coupling part (17) toward chamber part (11), and a tapered hole part (17a), the diameter of which is small toward connecting hole (15c) and the diameter of which becomes larger farther from connecting hole (15c). An engaging hole (17b), the diameter of which is larger than the hole part (17a), is formed at the open side in the inside of coupling part (17). An engaging step part (17c) is formed on the outer circumferential surface of coupling part (17).

Female Luer part (18) is constituted with a tubular body of differing levels composed of base part (18a) that is coupled to coupling part (17), covering coupling part (17) and connecting part (18b) into which the male Luer part of another member (not shown) is inserted to connect the other member to the stopcock body (10). An engaging step part (18c) that can engage with engaging step part (17c) of coupling part (17) is formed on the inner circumferential surface of base part (18a), and female Luer part (18) can be removably attached to coupling part (17) by engaging step parts (17c) and (18c) with each other. Connecting part (18b) is also formed in a tubular shape narrower than base part (18a), and a tapered hole (18d) is formed inside, wherein the diameter of the portion toward base part (18a) is small and the diameter becomes larger farther from base part (18a).

A cylindrical engaging tube (18e) extends toward the opening in base part (18a) from the boundary between base part (18a) and connecting part (18b) in female Luer part (18). Engaging tube part (18e) is formed to a size that can engage in engaging hole (17b) of coupling part (17), and when engaging step parts (17c) and (18c) are engaged and female Luer part (18) is coupled to coupling part (17), engaging tube part (18e) engages in engaging hole (17b). When engaging tube part (18e) is engaged in engaging hole (17b), the edge at the opening of coupling part (17) goes inside the space between the inner circumferential surface of base part (18a) and the outer circumferential surface (18e) of engaging tube part (18e) in female Luer part (18).

An O-ring (19) made of rubber is arranged at the inside end of the space and the edge at the opening of coupling part (17) enters the aforementioned space compressing O-ring (19). The connection part between coupling part (17) and female Luer part (18) is sealed by O-ring (19). The abovementioned channel (14a) is also constituted by hole (17a) of coupling part (17) and the inside and hole (18d) of engaging tube part (18e). Female Luer part (18) constituted in this way is composed of a polypropylene molded body with outstanding chemical resistance, and the portions constituting stopcock body (10) other than the female Luer part (18) portion are constituted with a polycarbonate molded body that can be molded with high dimensional precision.

Valve body (20) is constituted with valve body (21) that is basically cylindrical and actuating part (22) that is coupled to the bottom end of valve body (21). Valve body (21) is arranged inside chamber (11) and can rotate around the axis of chamber (11). That is, a sliding projection (21 a) that can slide between the inner circumferential surface and engaging part (11 b) of chamber (11) is formed at the top end of valve body (21), and on the portion toward the bottom end on the outer circumferential surface of valve body (21), a sliding projection (21 b) that can engage so that it is capable of sliding in engaging groove (11c) of chamber (11) is formed circumferentially.

Valve body (21) is arranged so as not to come out of chamber (11) by engaging sliding projection (21a) between the inner circumferential surface and engaging part (11b) of chamber (11) and also by engaging sliding projection (21b) in engaging groove (11c). A groove part (23) for connecting the branch tubes (12), (13) or (14) prescribed is also formed on the surface of valve body (21) in the portions corresponding to connecting holes (15a), (15b) and (15c). For example, with the state shown in Figure 3, branch tubes (12), (13) and (14) are all connected through chamber (11).

When valve body (21) is which can rotated a little clockwise from this state, branch tube (12) will be blocked from chamber (11) and branch tubes (13) and (14) remain connected through chamber (11). When valve body (21) is rotated a little counterclockwise from the state in Figure 3, branch tube (14) is blocked from chamber (11) and branch tubes (12) and (13) remain connected through chamber (11). In addition, when valve body (21) is rotated 180° from the state in Figure 3, branch tubes (12), (13) and (14) are all blocked from chamber (11).

Actuating part (22) has three actuating pieces (22a), (22b) and (22c) and the actuating pieces (22a), (22b) and (22c) are formed held at 90 degree angles corresponding to branch tubes (12), (13) and (14). Note that the arrows applied to actuating pieces (22a), (22b) and (22c) indicate channels. That is, the state in Figure 1 (c) corresponds to Figure 3 and shows branch tubes (12), (13) and (14) connected to each other through chamber (11). It also shows that when actuating part (22) is rotated 90° clockwise from the state in Figure 1 (c), branch tubes (13) and (14) are connected, and when actuating part (22) is rotated 90° counterclockwise, branch tubes (12) and (13) are connected.

In this constitution, when a prescribed liquid medicine is supplied to a patient's body (not shown), the back end of a transfusion tube (not shown) connected to a needle for puncturing the patient is connected to branch tube (12). A male Luer part furnished at the tip of a transfusion tube extending from a container that contains the liquid medicine to be supplied to the patient is connected to branch tube (14). Then the liquid medicine is supplied to the patient by sending the liquid medicine in the container toward the patient with the puncture needle inserted into the patient's body. In addition to liquid medicine supplied from a container, when other liquid medicine or the like is supplied to the patient, the needle of an injector or the like is passed through rubber plug (16) with the liquid medicine sucked up into the injector and the liquid medicine is injected into chamber (11) from branch tube (13).

In this case, female Luer part (18) is constituted with polypropylene, which has outstanding chemical resistance, so that it does not deteriorate because of the liquid medicine. For this reason, cracks and breaks in female Luer part (18) can be prevented from occurring, even when the male Luer part at the tip of a transfusion tube is inserted into female Luer part (18) and stress is applied to female Luer part (18). The portions that constitute stopcock body (10) other than female Luer pat (18) are constituted with polycarbonate, so a molded article with satisfactory dimensional precision is obtained.

With stopcock for medical treatment (A) pertaining to this embodiment, rather than the stopcock body being constituted with a monobloc resin molded body, female Luer part (18) which is connected to another member in branch tube (14) is constituted with a separate body from the other portions constituting stopcock body (10). By constituting female Luer body (18) with polypropylene, which has outstanding chemical resistance, cracks and breaks can be prevented from occurring. By constituting the portions constituting stopcock body (10) other than female Luer part (18) with polycarbonate, which can be molded with high dimensional precision, [the entire stopcock] is formed with high precision. Because an O-ring (19) is also furnished in the connection part between coupling part (17) and female Luer part (18) constituting branch tube (14), leakage from the connection part can be more reliably prevented from occurring. In addition, because the inside of branch tube (13) is closed with rubber plug (16) and blocks air getting into chamber (11), bacterial growth can be prevented. That is, all the space inside chamber (11) is a channel for liquid medicine or the like and no air will remain.

Figure 6 shows a stopcock for medical treatment (B) pertaining to another embodiment of the present invention. With stopcock for medical treatment (B), branch tube (33) corresponding to branch tube (13) of abovementioned stopcock for medical treatment (A) is formed smaller and longer than branch tube (13). Branch tube (33) is also formed with a polypropylene molded body that is removable from chamber part (31). The constitution of the other portions of stopcock for medical treatment (B) are the same as the corresponding portions of abovementioned stopcock for medical treatment (A), so the same symbols are noted for the same portions and the explanation is omitted. The operation and effects of stopcock for medical treatment (B) are also the same as for abovementioned stopcock for medical treatment (A).

The stopcock for medical treatment pertaining to the present invention is not limited to the abovementioned embodiments, and changes can be implemented as is appropriate. For example, with the abovementioned embodiments, branch tube (14) is constituted with tubular coupling part (17) and female Luer part (18), but coupling part (17) could be constituted with a projection that protrudes slightly from chamber part (31) and most of branch tube (14) could be constituted with the female Luer part. The female Luer part could also be coupled to the chamber part using a screw or the like. In addition, with the abovementioned application examples, an O-ring (19) made of rubber is used as the seal material, but the seal material can be constituted with a material other than rubber and other than an O-ring can be used. O-ring (19) can also be omitted as long as a liquid-tight seal can be maintained between coupling part (17) and base part (18a) of female Luer part (18). In addition, changes can also be implemented as is appropriate for the shape, materials, or the like of portions other than those constituting the stopcock for medical treatment.

## Claims

1. A valve unit (A, B) comprising a housing (10) having a plurality of connection ports (12, 13, 14) attached thereto, wherein at least one of the connection ports comprises a connector (18) removably attachable to said housing, **characterized in that** said connector is composed of a resin material more resistant to chemical attack than a material of said housing and that when said connector is attached to said housing, said connector provides a Luer connection for the attachment of a further Luer connector thereto.

2. The valve unit according to Claim 1, wherein said housing is composed of a resin material moldable to a higher precision than the material of said connector.

3. The valve unit according to Claim 1, wherein said connector is a female Luer connector.

4. The valve unit according to Claim 3, wherein said female Luer connector is connected to said housing by means of a snap connection.

5. The valve unit according to Claim 4, wherein said snap connection includes an O-ring seal (19).

6. The valve unit according to Claim 1, wherein said connector (18) is fabricated from polypropylene and the housing (10) is fabricated from polycarbonate.

7. The valve unit according to any preceding claim, wherein the valve unit is a stopcock for medical treatment.

## Patentansprüche

1. Ventileinheit (A, B), die ein Gehäuse (10) mit mehreren daran befestigten Verbindungsanschlüssen (12, 13, 14) umfasst, wobei mindestens einer der Verbindungsanschlüsse einen Verbinder (18) umfasst, der lösbar an dem Gehäuse befestigt werden kann, **dadurch gekennzeichnet, dass** der Verbinder aus einem Harzmaterial besteht, das resistenter gegen chemische Angriffe ist als ein Material des Gehäuses, und dass der Verbinder, wenn der Verbinder an dem Gehäuse befestigt ist, einen Luer-Anschluss zur Befestigung eines weiteren Luer-Verbinders daran bereitstellt.

2. Ventileinheit nach Anspruch 1, wobei das Gehäuse aus einem Harzmaterial besteht, das mit einer höheren Genauigkeit geformt werden kann als das Material des Verbinders.

3. Ventileinheit nach Anspruch 1, wobei der Verbinder eine Luer-Buchse ist.

4. Ventileinheit nach Anspruch 3, wobei die Luer-Buchse durch eine Schnappverbindung mit dem Gehäuse verbunden ist.

5. Ventileinheit nach Anspruch 4, wobei die Schnappverbindung eine O-Ring-Dichtung (19) enthält.

6. Ventileinheit nach Anspruch 1, wobei der Verbinder (18) aus Polypropylen und das Gehäuse (10) aus Polycarbonat hergestellt ist.

7. Ventileinheit nach einem vorhergehenden Anspruch, wobei die Ventileinheit ein Absperrhahn für eine medizinische Behandlung ist.

## Revendications

1. Unité de valve (A, B), comprenant un boîtier (10) ayant une pluralité d'orifices de connexion (12, 13, 14) attachés à celui-ci, au moins l'un des orifices de connexion comprenant un connecteur (18) pouvant être attaché de manière détachable audit boîtier, **caractérisée en ce que** ledit connecteur est composé d'un matériau à base de résine plus résistant aux attaques chimiques qu'un matériau dudit boîtier et **en ce que** lorsque ledit connecteur est attaché audit boîtier, ledit connecteur fournit un verrouillage de type Luer pour y attacher un connecteur Luer supplémentaire.

2. Unité de valve selon la revendication 1, dans laquelle ledit boîtier se compose d'un matériau à base de résine pouvant être moulé avec une plus grande précision que le matériau dudit connecteur.

3. Unité de valve selon la revendication 1, dans laquelle ledit connecteur est un connecteur de Luer femelle.

4. Unité de valve selon la revendication 3, dans laquelle ledit connecteur Luer femelle est connecté audit boîtier au moyen d'une connexion par encliquetage.

5. Unité de valve selon la revendication 4, dans laquelle ladite connexion par encliquetage comporte un joint torique (19).

6. Unité de valve selon la revendication 1, dans laquelle ledit connecteur (18) est fabriqué en polypropylène et le boîtier (10) est fabriqué en polycarbonate.

7. Unité de valve selon l'une quelconque des revendications précédentes, dans laquelle l'unité de valve est un robinet d'arrêt pour un traitement médical.
